(19)

## Europäisches Patentamt
## European Patent Office
## Office européen des brevets

(11) Veröffentlichungsnummer: **0 003 616**
**B1**

(12)

# EUROPÄISCHE PATENTSCHRIFT

(45) Veröffentlichungstag der Patentschrift: **24.02.82**

(21) Anmeldenummer: **79100441.9**

(22) Anmeldetag: **14.02.79**

(51) Int. Cl.³: **C 12 P 19/28,**
**C 07 H 15/20, C 08 B 37/00**
**//A61K31/71**

(54) **Neue Aminozucker-Derivate, ihre Herstellung und Amylase-hemmende Mittel.**

(30) Priorität: **14.02.78 GB 583478**

(43) Veröffentlichungstag der Anmeldung:
**22.08.79 Patentblatt 79/17**

(45) Bekanntmachung des Hinweises auf die Patenterteilung:
**24.02.82 Patentblatt 82/8**

(84) Benannte Vertragsstaaten:
**BE CH DE FR GB IT LU NL SE**

(56) Entgegenhaltungen:
FR - A - 2 120 073
FR - A - 2 244 536
FR - A - 2 346 368

(73) Patentinhaber: **F. HOFFMANN-LA ROCHE & CO.**
**Aktiengesellschaft**
**CH-4002 Basel (CH)**

(72) Erfinder: **Yasuji, Suhara**
**Dream Height 9-506, Matano-cho 1403-banchi**
**Totsuka-ku Yokohama-shi Kanagawa-ken (JP)**
Erfinder: **Kiyoshi, Ogawa**
**2222 Banchi Kameino**
**Fujisawa-shi Kanagawa-ken (JP)**
Erfinder: **Kazuteru, Yokose**
**Nekozane 981-2, Urayasu-machi**
**Higashi Katsushika-gun Chiba-ken (JP)**
Erfinder: **Kimihiro, Watanabe**
**Kamitsuchidana 2009-59 Ayase-machi**
**Koza-gun Kanagawa-ken (JP)**

(74) Vertreter: **Wuesthoff, Franz, Dr.-Ing. et al,**
**Patentanwälte Wuesthoff -v. Pechmann-Behrens-**
**Goetz Schweigerstrasse 2**
**D-8000 München 90 (DE)**

Courier Press, Leamington Spa, England.

**0 003 616**

Neue Aminozucker-Derivate, ihre Herstellung und Amylase-hemmende Mittel

Die vorliegende Erfindung betrifft neue Aminozucker-Derivate, die als gemeinsames strukturelles Merkmal Trehalose enthalten, und deren Salze.

Die erfindungsgemässen Aminozucker-Derivate werden nachfolgend "Trestatine" genannt.

Die Erfindung betrifft weiterhin ein Verfahren zur Herstellung der Trestatine und Amylase-hemmende Kompositionen, die ein oder mehrere Trestatine oder Salze davon enthalten.

Die Erfindung betrifft insbesondere Trestatin A, Trestatin B und Trestatin C, deren Salze und Gemische davon, wie auch Kompositionen die mindestens eines der Trestatine A, B oder C oder ein Salz enthalten.

a) Elementar-Analyse:

|  | C %  | H %  | N %  | O %  |
|---|---|---|---|---|
| Trestatin A: | 46,72 | 7,13 | 2,08 | 43,28 |
| Trestatin B: | 46,27 | 7,04 | 1,65 | 44,69 |
| Trestatin C: | 47,55 | 7,15 | 2,29 | 42,66 |

b) Molekulargewicht (Osmometrie):

| Trestatin A: | 1470 |
|---|---|
| Trestatin B: | 975 |
| Trestatin C: | 1890 |

c) Schmelzpunkt:

| Trestatin A: | 221—232°C (Zers.) |
|---|---|
| Trestatin B: | 209—219°C (Zers.) |
| Trestatin C: | 230—237°C (Zers.) |

d) Spezifische Drehung:

| Trestatin A: | $[\alpha]_D^{24} = +177°$ (c = 1,0, $H_2O$) |
|---|---|
| Trestatin B: | $[\alpha]_D^{26} = +187°$ (c = 1,0, $H_2O$) |
| Trestatin C: | $[\alpha]_D^{23} = +169,5°$ (c = 1,0, $H_2O$) |

e) Ultraviolett-Absorptionsspektrum (in Wasser):

Trestatin A, Trestatin B und Trestatin C zeigen Endabsorption.

f) Infrarot-Absorptionsspektrum (in KBr):

| Trestatin A: | siehe Figur 1 |
|---|---|
| Trestatin B: | siehe Figur 2 |
| Trestatin C: | siehe Figur 3 |

g) ¹H NMR-Spektrum (in $D_2O$ bei 100 MHz):

| Trestatin A: | siehe Figur 4 |
|---|---|
| Trestatin B: | siehe Figur 5 |
| Trestatin C: | siehe Figur 6 |

h) Löslichkeit in Lösungsmitteln:

Trestatin A, Trestatin B und Trestatin C und deren Hydrochloride sind leicht löslich in Wasser; löslich in Dimethylsulfoxyd; schwach löslich in Aethanol und Aceton; und unlöslich in Aethylacetat und Chloroform;

i) Farbreaktion:

Trestatin A, Trestatin B und Trestatin C geben eine positive Permanganat und Anthron-Reaktion und eine negative Sakaguchi-Reaktion.

Die neuen Trestatine, d.h. Trestatin A, Trestatin B und Trestatin C werden erfindungsgemäss dadurch hergestellt, dass man Streptomyces dimorphogenes NR—320—OM7HB (=ATCC 31484) oder NR—320—OM7HBS (=ATCC 31485) unter aeroben Bedingungen im wässrigem Medium kultiviert, die Trestatine aus der Fermentationsbrühe abtrennt und gewünschtenfalls Trestatin A, Trestatin B und Trestatin C voneinander trennt.

Die bei dem erfindungsgemässen Verfahren verwendeten Stämme des Genus Streptomyces sind auf einer Bodenprobe von Chichibushi, Saitama-ken, Japan, isoliert worden und werden zunächst bei der Agency of Industrial Science and Technology, Fermentations Research Institute, Japan, unter der Bezeichnung "FERM—P No. 3664" bzw. "FERM—P No. 3665" hinterlegt. Die Stämme wurden weiterhin bei der American Type Culture Collection (ATCC) Parklawn Drive, Rockville, Maryland 20852 (USA) am 30. Januar 1979 hinterlegt und haben die Nummern ATCC 31484 und ATCC 31485 erhalten.

Sie weisen die folgenden mykologischen Charakteristiken auf:

I. Morphologie

Mikroskopisch entwickelt FERM—P No. 3664 überwiegend gerades und selten spiralförmiges Luftmycel mit 10—50 Sporen pro Kette aus gut verzweigtem Mycel auf verschiedenen ISP-Medien. Die Sporen haben eine glatte Oberfläche, sie sind von 0,6—1,0 x 0,8—1,4 $\mu$ gross. Verzweigungen am spiraligen Mycel oder Sporangium-Bildung wurden nicht festgestellt. Weiterhin wurde bei der Beobachtung der Morphologie des Luftmycels eine kleine Population (weniger als 1%) mit spiraligem Luftmycel festgestellt, die als Cluster innerhalb einer dominierenden Population mit gerader Sporenkette auftrat. Bei der Einzelisolierung der spiraligen Kolonnie zeigte sich, dass der Clon seine Morphologie des spiraligen Luftmycels über Generationen beibehielt, wobei konstant ein kleiner Teil (0,7—2%) gerades Luftmycel als Büschel oder Kolonien produziert wurde. Diese durch Einzelisolierung der spiraligen Kolonie aus dem Stamm FERM—P 3664 erhaltene Subspecies wird als FERM—P 3665 bezeichnet. Ein deutlicher Unterschied in den Charakteristiken von FERM—P 3664 und FERM—P 3665 wurde, abgesehen von der Morphologie des jeweiligen Luftmycels, nicht festgestellt.

II. Kultur-Charakteristiken

Die Stämme FERM—P 3664 und FERM—P 3665 haben die gleichen, in Tabelle I aufgeführten Kultur-Charakteristiken. Alle Tests wurden mit bei 27°C gewachsenen Kulturen ausgeführt mit Ausnahme des Magermilchtests, der bei 37°C durchgeführt wurde. Farbbestimmungen wurden mit 14—21 Tage alten Kulturen anhand des Color Harmony Manual, 4th ed. 1958 (Container Cooperation of America, Chicago) ausgeführt.

TABEL·LE 1':

| Medium | Aeriales Mycel | Substrat Mycel | Revers-Substrat Mycel | Lösliches Pigment |
|---|---|---|---|---|
| Sucrose-Nitrat Agar | bräunlich-weiss — hellgrau (3dc, Natural) | gut, hellgelblich braun — gräulich hellbraun (31g, Lt Brown) | gelblich braun (3ng, Yellow maple) — stark braun (4pl, Deep Brown) | bräunlich |
| Glucose; Asparagin-Agar | dunn, bräunlich grau | mässig, hellgelblich braun (21e, Old Gold) — matt gelblich orange | hell gelblich braun (21e, Old Gold) — matt gelblich orange | keines |
| Glycerin- Asparagin-Agar (ISP med. 5) | gräulich weiss — hellgrau (2dc, Natural — 2fe, Covert Gray) | gut, gräulich gelb braun — gelblich braun (3ng, Yellow Maple) | gräulich gelb braun — dunkel gelblich braun (3ni, Clove Brown) | braun |
| Anorganische Salze-Stärke- Agar (ISP med. 4) | hellgrau (2fe, Covert Gray) — hell bräunlich grau (3fe, Silver Gray) | gut, gelblich braun — braun (31g, Cinnamon Brown) | gelblich braun — braun (31g, Cinnamon Brown) | braun |
| Tyrosin-Agar (ISP med. 7) | hellgrau (2dc, Natural — 2fe, Covert Gray) — grau | gut, hellbraun — gelblich braun (3ng, Yellow Maple) | hellbraun — gelblich braun (3ng, Yellow Maple) | schwach bräunlich |
| Nähr-Agar | dünn, weiss | schwach, hellgelblich bräun | farblos — hell gelblich braun | keines |
| Hefeextrakt- Maisextrakt- Agar (ISP med. 2) | hellbrau (2fe, Covert Gray) — hell bräun- lich grau (3fe, Silver Gray) | mässig — gut, gräulich gelb-braun — braun (31g, Cinnamon Brown) | gräulich gelb braun — braun (31g, Cinnamon Brown) | schwach bräunlich |
| Haferschleim- Agar (ISP med. 3) | hellgrau (2fe, Covert Gray) — hell bräunlich grau (3fe, Silver Gray) | gut, hellgelb — hell gelblich braun (2gc, Bamboo — 2ie, Lt Mustard Tan) | hellbraun — hell gelblich braun (2gc, Bamboo — 2ie, Lt Mustard Tan) | gelblich |
| Magermilch- Nahr-Agar | keines | farblos — hellgelb | farblos — hellgelb | keines |

TABLELLE 1: Fortsetzung

| Medium | Aeriales Mycel | Substrat Mycel | Revers-Substrat Mycel | Lösliches Pigment |
|---|---|---|---|---|
| Glucose-Pepton-Gelatine-Ansatz | keines | farblos — hellgelb | — | keines |
| Magermilch (37°C) | dünn, weiss | bräunlich grau — hellbraun | — | keines |

0003616

TABELLE II: Vergleich morphologischer, kulturmassiger und physiologischer Charakteristiken der Stamme NR–320–OM7HB, NR–320–OM7HBS und verwandter Stamme

| | NR–320–OM7HB | NR–320–OM7HBS | S. nigrifaciens ISP 5071 | S. olivaceus ISP 5072 | S. plicatus ISP 5319 |
|---|---|---|---|---|---|
| Morphologisches Sektion (ISP med. 2,3,4,5) | RF[a], (S) | S[b], (RF) | RF | S, RA[c], RF | S, RA |
| Sporenoberflache | glatt | glatt | glatt | glatt | glatt |
| **ISP Medium 3** | | | | | |
| a.m. [d] | reichlich | reichlich | reichlich | dünn | mässig, ausgebreitet |
| | hellbrau — hell bräunlich grau | hellgrau — hellbraunlich grau | olivgrau | hell braunlich grau | hell bräunlich grau |
| s.m. [e] | hellgelb — hell gelblich braun | hellgelb — hell gelblich braun | gräunlich gelb — dunkelgelb | hell gelblich braun | grünlich gelb braun |
| r.s.m.[b] | hellgelb — hell gelblich braun | hellgelb — hell gelblich braun | mattgelb | farblos — hell gelblich braun | hell gelblich braun |
| s.p. [g] | gelblich | gelblich | gelb | keines | keines |
| **ISP Medium 4** | | | | | |
| a.m. | reichlich | reichlich | reichlich | dünn | mässig, ausgebreitet |
| | hellgrau — hell bräunlich grau | hellgrau — hell bräunlich grau | hell bräunlich grau | hell bräunlich grau | hell bräunlich grau |
| s.m. | gelblich braun — braun | gelblich braun — braun | dunkelgelb — gelblich braun | hell gelblich braun — dunkel gelblich braun | gelblich braun — dunkel gelblich braun |
| r.s.m. | braun | braun | gelblich braun | hellgelb — hell gelblich braun | gelblich braun |
| s.p. | braun | braun | braun | keines | keines |

TABELLE 2: Fortsetzung

| | NR–320–OM7HB | NR–320–OM7HBS | S. nigrifaciens ISP 5071 | S. olivaceus ISP 5072 | S. plicatus ISP 5319 |
|---|---|---|---|---|---|
| Melanin-Bildung [A] | | | | | |
| auf ISP Medium 1 | (−) | (−) | − | − | − |
| auf ISP Medium 6 | − | − | − | − | − |
| auf ISP Medium 7 | (−) | (−) | − | − | − |
| Kohlenstoff-Verwertung [B] | | | | | |
| L-Arabinose | ++ | ++ | + | ++ | ++ |
| D-Xylose | ++ | ++ | + | + | ++ |
| D-Glucose | ++ | ++ | ++ | ++ | ++ |
| D-Fructose | ++ | ++ | ± | + | ++ |
| Sucrose | ++ | ++ | − | − | − |
| I-Inosit | ++ | ++ | − | ± | ++ |
| Rhamnose | ++ | ++ | ++ | ++ | ++ |
| Raffinose | ++ | ++ | − | − | − |
| D-Mannit | ++ | ++ | ++ | ++ | ++ |
| Casein-Hydrolyse [C] | + | + | + | + | + |
| Starke-Hydrolyse | + | + | + | + | + |
| Gelatine-Verflüssigung | + | + | + | + | − |
| Nitrat-Reduktion | − | − | − | − | − |
| Milch-Koagulation | − | − | + | − | + |
| Milch-Peptonisierung | + | + | + | + | + |
| Temperaturbereich des Wachstums auf ISP–2 (°C) | 20–45 | 20–45 | 20–37 | 20–37 | 20–45 |

a; rectiflexibiles     b; spirales     c; retinaculiaperti     d; aeriales Mycel     e; Substrat Mycel
f; revers-Substrat-     g; lösliches Pigment
   Mycel
A) −; nicht gebildet,     (−); vermutlich nicht gebildet
B) ++; gute Verwertung,     +; Verwertung,     ±; fragliche Verwertung,     −; keine Verwertung
C) +; positiv,     −; negativ

**0 003 616**

III. Physiologische Eigenschaften

Kein deutlicher Unterschied wurde in den nachstehenden Charakteristiken zwischen den Stämmen FERM—P No. 3664 und 3665 festgestellt.

1) Temperaturbereich des Wachstums

Auf ISP-2-Medium wurde mässiges bis reichliches Wachstum bei 20°, 25°, 27°, 30°, 37° und 45°C beobachtet, jedoch nicht bei 6°C und 55°C. Als optimale Wachstumstemperatur wurde etwa 37°C festgestellt.

2) Gelatineverflüssigung auf Glucose-Pepton-Gelatine-Agar bei 27°C.

Eine Verflüssigung mässiger Intensität trat um den 12. Tag ein.

3) Stärkehydrolyse auf anorganische Salz-Stärke-Agar bei 27°C.

Es trat eine Hydrolyse mittlerer Intensität auf.

4) Koagulation und Peptonisierung in 10% Magermilchmedium bei 37°C.

Eine Peptonisierung starker Intensität wurde um den 5. Tag beobachtet. Die Koagulation war negativ.

5) Caseinhydrolyse auf 10% Magermilch-Nähragar bei 27°C.

Es wurde eine Hydrolyse mässiger bis starker Intensität festgestellt.

6) Nitratreduktion auf ISP-8-Medium bei 27°C.

Negativ.

7) Melaninbildung bei 27°C.

Auf Pepton-Hefe-Eisenagar (ISP-6) wird kein Pigment gebildet. Die Pigmentbuildung wurde auch auf Trypton-Hefebrühe (ISP-1) und Tyrosin-Agar (ISP-7) negativ beurteilt.

8) Kohlenhydratverwertung auf Pridham-Gottlieb-Agar-medium (ISP-9) bei 27°C.

Reichliches Wachstum wurde mit L-Arabinose, D-Xylose, D-Glucose, D-Fructose, Sucrose, Inosit, Raffinose, D-Mannit und L-Rhamnose beobachtet.

Zusammenfassend können die oben erwähnten mikrobiologischen Eigenschaften der Stämme FERM—P No. 3664 und 3665 wie folgt beschrieben werden:

Die Stämme sind Actinomycetales und gehören zum Genus Streptomyces. Die Form des Luftmycels ist gerade und spiralig (überwiegend gerade bei NR—320—OM7HB und überwiegend spiralig bei NR—320—OM7HBS). Quirlbildung wurde nicht bemerkt. Die Sporen beider Stämme sind oberflächlich glatt und 0,6—1,8 × 0,8—1,4 $\mu$ gross. Die Stämme entwickeln auf verschiedenen ISP Medien hellgraues bis hellbräunlich-graues Luftmycel von hellgelblich-braunem bis gelblich-braunem Wachstum mit bräunlichem löslichen Pigment. Melanoides Pigment wurde auf Pepton-Hefe-Eisen-Agar nicht und auf Trypton-Hefebrühe und Tyrosin-Agar wahrscheinlich nicht gebildet. Die Stämme peptonisierten Magermilch stark. Die Gelatineverflüssigung war schwach, während Stärkehydrolyse im mässigen Ausmass auftrat. In allen wie oben ausgeführten Tests zeigte sich keinen Unterschied zwischen NR—320—OM7HBS und NR—320—OM7HB.

Diese Charakteristiken legen nahe, dass die Stämme dem S. nigrifaciens ähneln. Der Vergleich der vorliegenden Stämme mit den ISP-Stämmen ist aus der Tabelle II ersichtlich. Es zeigte sich, dass die beiden Stämme, nämlich NR—320—OM7HB und NR—320—OM6HBS von jeder dieser drei vorstehend genannten, nahe verwandten Spezies unterscheidbar sind.

Im Vergleich mit S. nigrifacies unterscheidet sich NR—320—OM7HB von ersteren bei der Peptonisierung von Magermilch und der Verwertung von Sucrose, Inosit und Raffinose. Morphologisch bildete S. nigrifacies niemals Spiralen. Diese Tatsachen, zusammen mit der Produktion eines olivgrauen Luftmycels, grünlichgelbem Substratmycel auf ISP-3-Medium und Unfähigkeit zur Inhibiterproduktion machen es unwahrscheinlich, dass beide Stämme eine identische Spezies bilden.

Andererseits erscheint die Sporenkette von S. plicatus ISP 5319 hauptsächlich in spiraliger Form auf ISP-2-, 3-, 4- und 5-Medien. Obgleich schlingenförmiges Luftmycel weniger häufig bemerkt wurde, wurde keine gerade Form bei ISP 5319 gesehen, die S. plicatus von NR—320—OM7HB unterscheidet. Weiterhin wurden grundlegende Unterschiede in der Ausbreitung des Luftmycels, der Pigmentierung, der Koagulierung, der Gelatineverflüssigung, und der Verwertung von Sucrose und Raffinose gesehen.

S. olivaceus zeigte sich unter den drei geprüften Spezies dem NR—320—OM7HB am nächsten verwandt. So zeigte S. olivaceus ISP 5072 spiralige Ausbildung in den meisten Teilen auf ISP-2-, 3-, 4- und 5-Medien, teilweise mit schlingenförmig-geraden Sporenketten am Luftmycel. Er unterscheidet sich jedoch sowohl von NR—320—OM7HB als auch von NR—320—OM7HBS darin, dass spiralförmige, schlingenförmige und gerade Sporenketten innerhalb eines Clusters Luftmycel nebeneinander existieren, was bei NR—320—OM7HB und NR—320—OM7HBS neimals beobachtet wurde. Zwischen ISP 5072 und NR—320—OM7HB wurden weiterhin Unterschiede beobachtet bei der Produktion von löslichem Pigment (keine Pigmentierung bei ISP 5072 auf ISP-2-, 3-, 4- und 5-Medien), Entwicklung von Luftmycels (dünnes Luftmycel bei ISP 5072) Verwertung der Kohlenstoffquelle (Sucrose/Raffinose) und Produktion von $\alpha$-Amylase-Inhibitor (keine Hemmung durch ISP 5072-Metaboliten). Alle diese Resultate zeigten, dass NR—320—OM7HB wie auch NR—320—OM7HBS am nächsten verwandt, aber nicht identisch, mit der Spezies S. olivaceus ISP 5072 zu sein scheinen.

Es erscheint daher berechtigt, dieses Taxon als eine neue Spezies des Genus Streptomyces an-

8

zusehen sind diese Spezies wird hier als Streptomyces dimorphogenes nov. sp. WATANABE und MARUYAMA, 1978 beizeichnet. Der Typenstamm von S. dimorphogenes ist NR—320—OM7HB (FERM—P No. 3664). Die Etymologie basiert auf dem Vorliegen zweier Formen in der Morphologie der Sporenkette, d.h. der stellenweisen Verteilung spiralkettiger Büschel als Minorität. Ein Biotyp, NR—320—OM7HBS (FERM—P No. 3665) in dem die spiralförmige Sporenkette dominant ist, mit stellenweiser Verteilung von geraden Sporenketten als kleinere Cluster wäre dann zweckmässig als S. dimorphogenes subsp. spiroformatus zu benennen, unter Bezugnahme auf die Morphologie des Luftmycels. Der Typenstamm FERM—P No. 3664 sollte dann entsprechend als S. dimorphogenes subsp. dimorphogenes benannt werden.

Die Trestatine können erfindungsgemäss dadurch erhalten werden, dass man einen Trestatinproduzierenden Mikroorganismus des Genus Streptomyces, z.B. Streptomyces dimorphogenes sp. nov., Watanabe & Maruyama (NR—320—OM7HB FERM—P No. 3664) oder Streptomyces dimphorphenes subsp. spiroformatus (NR—320—OM7HBS, FERM—P No. 3665) unter aeroben Bedingungen in einem wässrigen Medium kultiviert.

Die Kultivierung kann in einem Kulturmedium durchgeführt werden, dass übliche Nährsubstanzen, dieser Mikroorganismus verwenden kann, enthält. Kohlenstoffquellen sind beispielsweise Stärke, Dextrin, Glucose, Glycerin, Sucrose, Trehalose, Melasse oder Gemische davon; Stickstoffquellen sind z.B. Sojabohnenpulver, Fleischextrakt, Pepton, Hefeextrakt, Maisquellwasser, Ammoniumsulfat, Natriumnitrat, Ammoniumchlorid oder Gemische davon. Das Kulturmedium kann falls erforderlich geeignete anorganische Substanzen wie Calciumcarbonat, Natriumchlorid und/oder Salze von Zink, Kupfer, Mangan oder Eisen enthalten. Zusätzlich kann ein Antischaummittel wie Silikon oder vegetative Oele zugesetzt werden, um eine Schäumen während des Kultivierens zu verhüten.

Die Kultivierung kann unter aeroben Bedingungen in einem wässrigen Medium, insbesondere durch Submersfermentation vorgenommen werden. Das pH des Mediums zu Beginn der Kultivierung beträgt etwa 7. Die bevorzugte Temperatur liegt im Bereich von 20—40°, insbesondere von 25—30°C.

Nach Kultivierung während 1—5 Tagen unter den oben erwähnten Bedingungen erhalten werden. Die Kultivierung wird zweckmässig zu der Zeit abgebrochen, bei der die grösste Wirksamkeit der Trestatine erreicht ist. Die Menge der erhaltenen Trestatine kann nach der folgenden Methode bestimmt werden:

*Bestimmung der Trestatine*

Die Trestatine zeigen eine starke Hemmwirkung auf $\alpha$-Amylase. Sie können infolgedessen mittels einer Methode bestimmt werden, die den $\alpha$-Amylase-Test von P. Bernfeld (Methods in Enzymology, I, 149, 1955) verwendet.

Zwei Einheiten (eine Einheit $\alpha$-Amylase ist diejenige Enzymmenge, die unter den nachstehenden Bedingungen in Abwesenheit von Trestatin die Bildung von reduzierenden Zuckern in einer 1 m$\mu$ Mol Maltose äquivalenten Menge katalysiert) Schweinepankreas-$\alpha$-Amylase in 0,1 ml 6,4 mM Ammoniumsulfatlösung werden zu 0,6 ml 20 mM Phosphatpuffer (pH 6,9) gegeben, der 10 mM Natriumchlorid und verschiedene Mengen Trestatin enthält. Das Gemisch wird nach 5 Minutem langen Aequilibrieren bei 37°C, gefolgt von einem Zusatz von 0,5 ml 4%iger Stärkelösung 5 Minuten lang bei 37°C inkubiert. Die Reaktion wird durch Zusatz von 2 ml Dinitrosalicylsäurereagens (hergestellt nach P. Bernfeld) beendet. Das Gemisch wird 5 Minuten auf dem siedenden Wasserbad erhitzt und dann mit 10 ml Wasser verdünnt. Die Absorption ($A_1$) der erhaltenen gefärbten Lösung wird bei 540 nm gemessen, worauf die Aktivität der $\alpha$-Amylasehemmung nach der folgenden Gleichung berechnet werden kann:

$$\text{Hemmung} = \left(1 - \frac{A_1 - A_0}{A_2 - A_0}\right) \times 100 \ (\%)$$

$A_0$ Absorption ohne Enzym
$A_2$ Absorption ohne Trestatin

Die Hemmeinheit (IU) wird als diejenige Menge Trestatin definiert, die unter den oben beschriebenen Testbedingungen eine 50%ige Enzymhemmung verursacht.

Nach der Fermentation kann die Isolierung der erzeugten Trestatine aus der Fermentationslösung mit an sich bekannten Methoden, beispielsweise auf folgende Weise bewerkstelligt werden: Das Mycel wird durch Zentrifugieren oder Filtrieren abgetrennt. Die Trestatine sind wasserlösliche, schwach basische Verbindungen. Infolgedessen können zur Isolierung und Reinigung der Trestatine konventionelle Methoden angewandt werden, die für die Isolierung und Reinigung einer wasserlöslichen basischen Substanz angewandt werden. Beispielsweise kann dies durch Adsorptionsmethoden mit einem Absorptionsmittel wie Aktivkohle, oder Kationenauscherharzen; oder Fällungsmethoden mit einem Lösungsmittel wie einem Alkohol oder Aceton; oder chromatographischen Methoden unter Verwendung von Cellulose oder Sephadex oder mit Kombinationen der vorstehend erwähnten Methoden erreicht werden.

Die Isolierung von Trestatin A, Trestatin B und Trestatin C als Einzelverbindung aus dem Fermentationsfiltrat können zweckmässig durch Säulenchromatographie unter Verwendung eines Gemisches der H-Form und der Ammonium-Form eines schwach sauren Kationenaustauscherharzes durchgeführt werden. Eine vorteilhafte Methode ist die folgende:

Das pH des Fermentationsfiltrates wird mit Alkali wie Natriumhydroxyd, Kaliumhydroxyd, Natriumcarbonat oder Kaliumcarbonat auf pH 7 eingestellt. Das Filtrat wird dann mit Aktivkohle versetzt und die Lösung filtriert. Die Trestatine werden von der Kohle mit 30—70%, vorzugsweise 50%igem wässrigen Aceton eluiert. Das Elutionsmittel wird vorzugsweise mit einer Säure auf pH 1—3 eingestellt und die Elution wird bei 50—70°C ausgeführt. Das Eluat wird konzentriert und das Konzentrat über eine Kationenaustauscherharz-Säule wie Dowex 50 (H-Form) gegeben. Die Säule wird mit Wasser gewaschen und mit 1 N wässrigem Ammoniak eluiert. Die Fraktionen mit Amylase-Hemmaktivität werden gesammelt, unter vermindertem Druck konzentriert und lyophilisiert, wobei ein pulverförmiges Rohprodukt, welches die Trestatine enthält, erhalten wird. Das Rohprodukt wird mit Methanol zur Entfernung methanollöslicher Verunreinigungen behandelt. Die Trestatine sind fast unlöslich in Methanol. Der Methanol-unlösliche Rückstand mit den Trestatinen wird in Wasser gelöst. Die Lösung wird durch eine Anionenaustauscherharz-Säule wie Dowex 1 (Acetat-Form) gegeben. Die Säule wird mit Wasser entwickelt und das Eluat fraktioniert. Die aktive Fraktionen werden gesammelt, unter vermindertem Druck konzentriert und über eine Kationenauscherharz-Säule wie Dowex 50 (Ammonium-Form) gegeben. Die Säule wird mit Wasser eluiert. Die Fraktionen mit Amylase-Hemmaktivität werden gesammelt, konzentriert und lyophilisiert, wobei ein Pulver erhalten wird, welches wie die Hochdruckflüssig-Chromatographie zeigt, hauptsächlich aus Trestatin A, Trestatin B und Trestatin C bestehen. Die wässrigen Lösungen der genannten Trestatine werden über eine Kationenaustauscherharz-Säule wie Amberlit CG 50 (Gemisch der H-Form und der Ammonium-Form) gegeben. Das bevorzugte Verhältnis von H-Form zu Ammonium-Form leigt im Bereich von 1:9—9:1 (Volumenteile). Die Säule wird mit Wasser eluiert, wobei Trestatin B, Trestatin A und Trestatin C in der angegebenen Reihenfolge erhalten werden. Jedes so erhaltene Trestatin wird rechromatographiert, wobei die reinen Trestatine A, B und C erhalten werden. Diese Trestatine können gewünschtenfalls in verschiedene Salze wie die Hydrochloride, Sulfate, Phosphate, Acetate, Oxalate übergeführt werden.

Schmelzpunkt der Hydrochloride:

Trestatin A-hydrochlorid:
163—190°C (Zersetzung unter Schäumen)

Trestatin B-hydrochlorid:
158—186°C (Zersetzung unter Schäumen)

Trestatin C-hydrochlorid:
190—200°C (Zersetzung unter Schäumen)

Spezifische Drehung der Hydrochloride:

Trestatin A-hydrochlorid:
$[\alpha]_D^{25} = +162,5°$ (c = 1,0, 0,01N HCl)

Trestatin B-hydrochlorid:
$[\alpha]_D^{26} = +169°$ (c = 1,0, 0,01N HCl)

Trestatin C-hydrochlorid:
$[\alpha]_D^{25} = +160,5°$ (c = 1,0, 0,01N HCl)

Tritration (in Wasser):

|  | pKa' | Aequivalent |
|---|---|---|
| Trestatin A | 5,0 | 720 |
| Trestatin B | 5,0 | 960 |
| Trestatin C | 5,0 | 650 |

Diese Daten, verbunden mit dem oben angegebenen Molekulargewicht zeigen, dass Trestatin A, B und C zweisäurige, einsäurige bzw. dreisäurige Basen sind.

**0 003 616**

Empirische Formel:

Trestatin A: $C_{56}H_{94}N_2O_{40}$

Trestatin B: $C_{37}H_{63}NO_{28}$

Trestatin C: $C_{75}H_{125}N_3O_{52}$

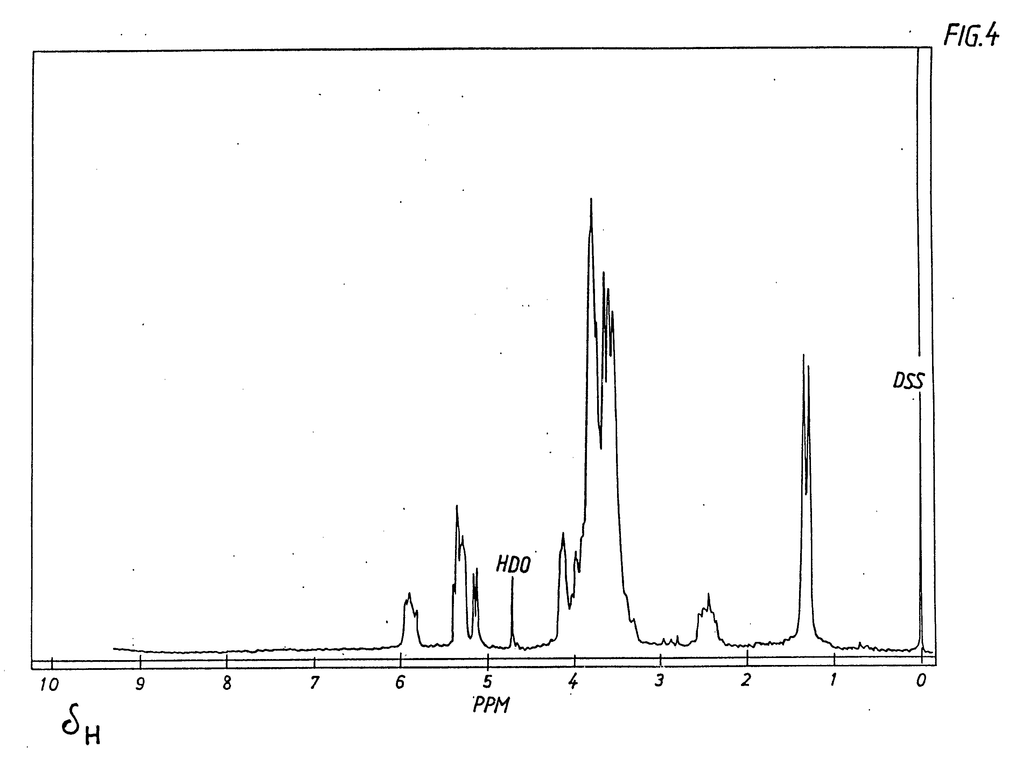

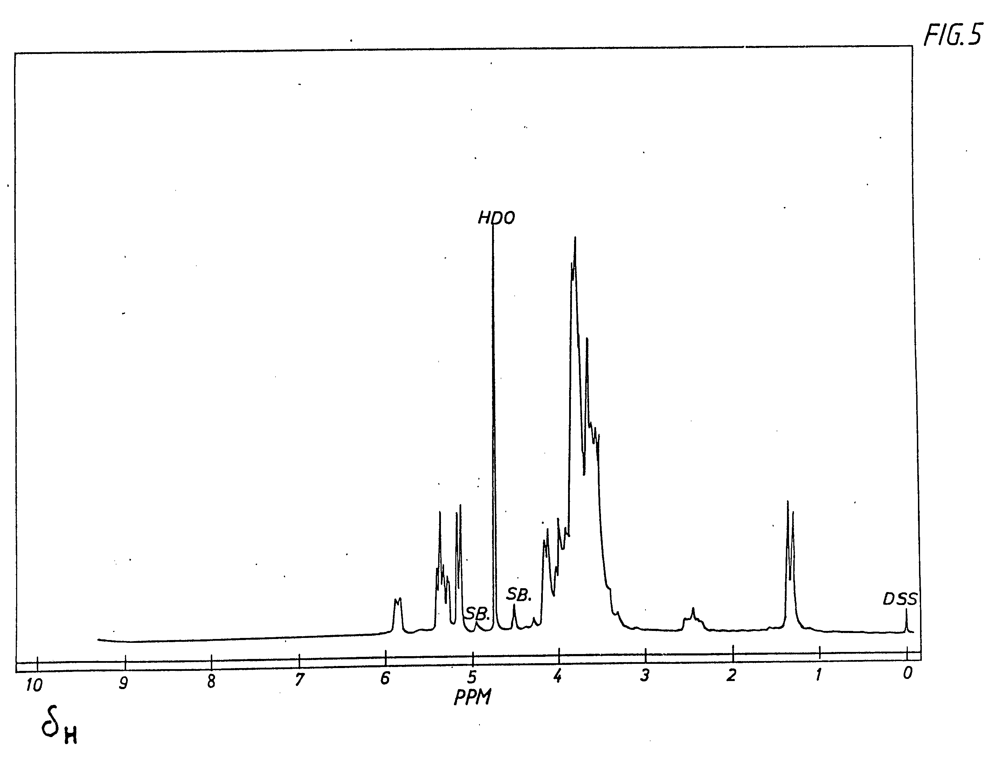

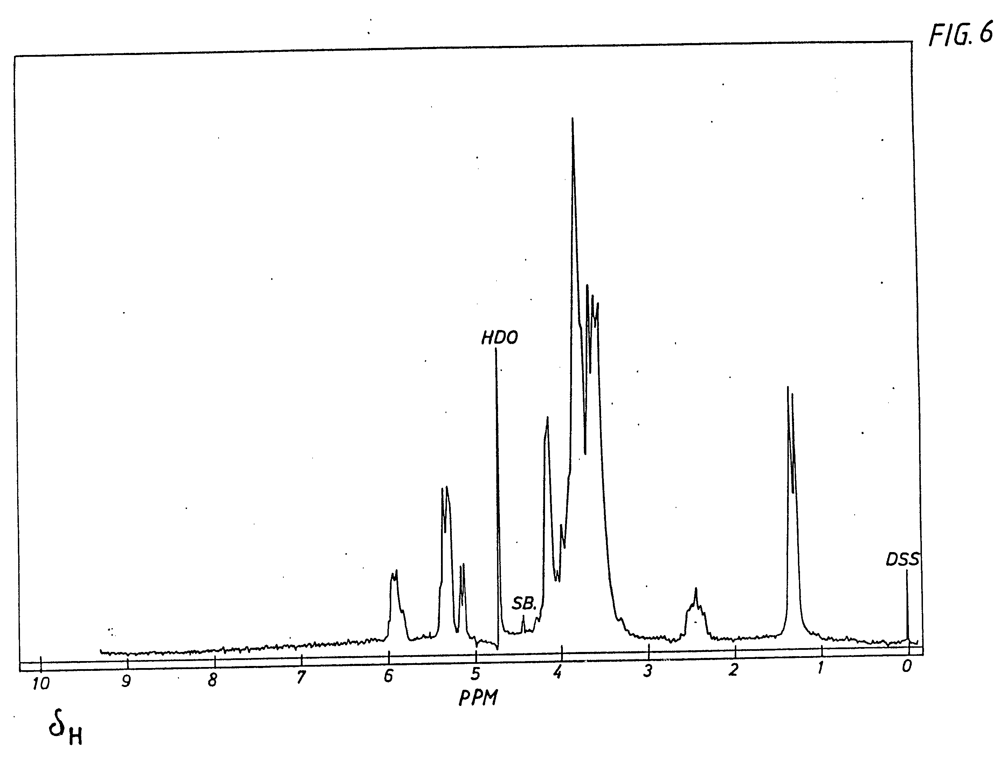

Die Kernresonanzspektren (in $D_2O$ bei 100 MHz) der Trestatine A, B, und C als freie Base wurden mit einem JEOL FX—100 Spektrometer mit "homo gated decoupling" (Einstrahlung bei $\delta$ 4,71 ppm) unter Verwendung von DSS als internem Standard aufgenommen und sind in Figur 4, 5 und 6 angegeben.

Dünnschichtchromatographie:

Platte: Silikagel $F_{254}$ (Merck)

Lösungsmittelsystem: Chloroform-Methanol 25% wässriges
Ammoniak-Wasser (1:4:2:1)

Nachweis: heisse Schwefelsäure

Unter den oben beschriebenen Bedingungen sind die Rf-Werte der Trestatine A, B und C 0,19, 0,28 bzw. 0,14.

Hochdruckflüssig-Chromatographie:

Säule: $\mu$-Bondapak/CH (1 feet × 0,25 inch, Waters Co., USA)

Lösungsmittel: Acetonitril-Wasser (63:37)

Fliessgeschwindigkeit: 4,0 ml/Min.

Nachweis: UV-Absorption bei 210 nm

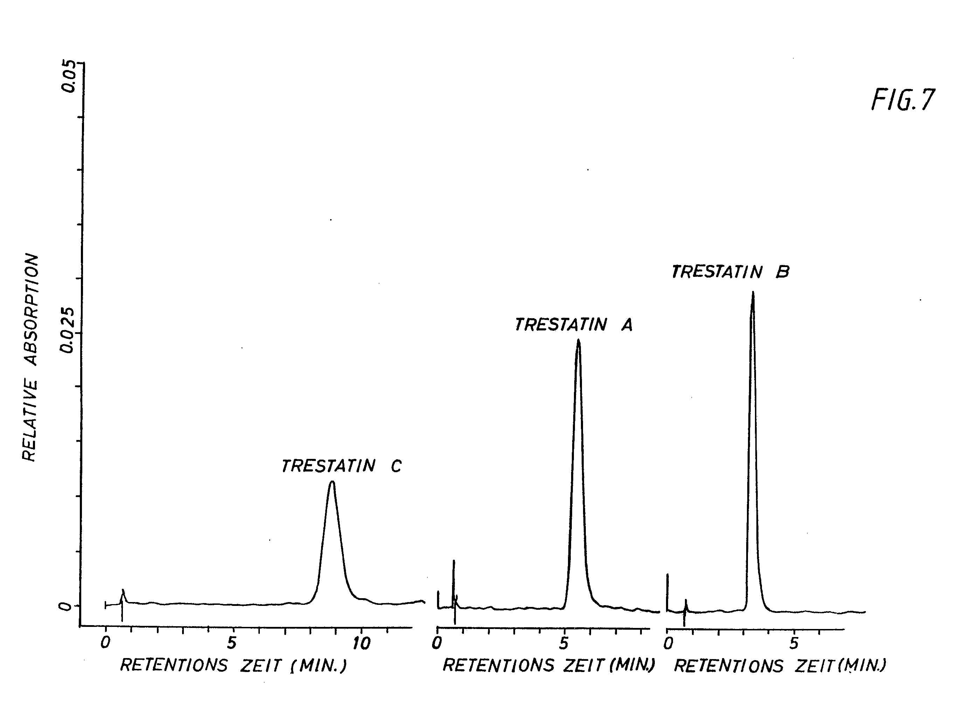

Unter den oben beschriebenen Bedingungen betragen die Retentionszeiten der Trestatine A, B und C 5,5, 3,4 bzw. 8,8 Min. Die Chromatrogramme finden sich in Figur 7.

Papierelektrophorese:

Papier: Toyo Roshi No. 51

Puffer: Ameisensäure-Essigsäure-Wasser (25:75:900, pH 1,8)

Spannung: 3000 V

Zeit: 40 Minuten

Unter den oben beschriebenen Bedingungen wandern alle Trestatine zur Kathode: Trestatin A: 12 cm, Trestatin B: 9 cm, Trestatin C: 13 cm.

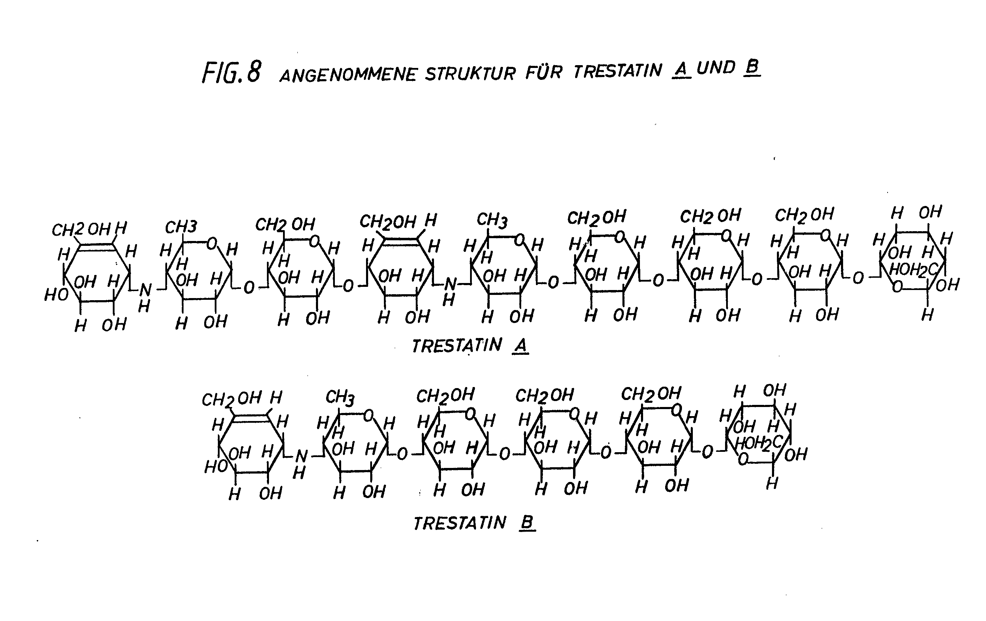

Struktur:
Auf Hydrolyse mit 4N Salzsäure bei 80°C während 3 Stunden liefern die Trestatine A, B und C Glucose und ein Amin. Das Amin hat ein pKa' von 3,9 und die Molekularformel $C_{13}H_{21}NO_7$, was sich aus dem "high resolution" Massenspektrum ergibt. Die Trestatine A, B und C enthalten 5,4 und 6 Mole Glucose. Milde Säurehydrolyse (80°C, 4 Stunden) mit Dowex 50 (H-Form als Katalysator Trehalose ($\alpha$-D-Glucopyranosyl-$\alpha$-D-glucopyranosid). Keines der Trestatine A, B und C enthält Aminosäuren.
Aus den oben erwähnten Eigenschaften ist abzuleiten, dass die erfindungsgemässen Trestatine A, B und C schwach basische Aminoglucoside mit Trehalose als gemeinsamen strukturellen Bestandteil sind. Anhand der obigen Befunde werden für die Trestatine A und B die in Figur 8 angegebenen Strukturen vorgeschlagen.
Trestatin A, Trestatin B und Trestatin C weisen eine hohe Amylase-Hemmaktivität auf, wie nachstehend gezeigt wird, und können als Amylase-Hemmer verwendet werden.

11

0 003 616

Konventionelle Amylase-Hemmer, die auf fermentativem Wege erzeugt werden, sind die folgenden:

1) Nojirimycin, Niwa et al. (Agr. Biol. Chem., *34*, 966, 1970)
Nojirimycin ist ein Monosaccharid und infolgedessen eindeutig verschieden von den erfindungsgemässen Trestatinen.

2) Peptidzucker, S. Ueda et al. (Agr. Biol. Chem., *37*, 2025, 1973 und Agr. Biol. Chem., *40*, 1167, 1976)
Der Peptidzucker enthält Aminosäuren als strukturellen Baustein.

3) Amylostatin A, S. Murao et al. (Japanese patent application, Kokai 123891/1975)
In der erwähnten Patentanmeldung wird beschrieben, dass Amylostatin A von stark sauren Kationenaustauscherharz im Bereich von pH 1—14 nicht absorbiert wird. Wie erwähnt, werden die Trestatine von einem solchen Ionenaustauscherharz adsorbiert.

4) Amylase-Inhibitor, K. Ueda et al. (Japanese patent application, Kokai 54990/1976)
Dieser Amylase-Hemmer hat ein Molekulargewicht von etwa 600, das von dem der Trestatine A, B und C verschieden ist.

5) Aminozucker-Verbindungen, W. Frommer et al., DE—A—2 347 782)
Diese Verbindungen sind einsäurige Basen während Trestatin A eine zweisäurige Base und Trestatin C eine dreisäurige Base ist. Trestatin B, obschon eine einsäurige Base, weist Trehalose als strukturellen Baustein auf und ist somit von den Aminozuckern von Frommer et al. verschieden.
Amylase-Hemmer mit Trehalose-Bausteinen sind bisher nicht beschrieben worden.
Die biologischen Eigenschaften der Trestatine und deren Salze sind die folgenden:

1) Akute Toxizität:
Eine akute Toxizität wird nicht beobachtet, selbst wenn 500 mg/kg Trestatin A, B und C oral an Ratten gefüttert werden (das ist das 65—700-fache der wirksamen Dosis). Ein Trestatin-Gemisch entsprechend dem in Beispiel 1 als Stufe III-Pulver bezeichneten Präparat wird in täglichen Dosierungen bis zu 8000 mg/kg während 10 Tagen von Mäusen und Ratten gut vertragen.

2) Antimikrobielles Spektrum:
Das Fehlen antimikrobieller Aktivität von Trestatin A, B und C, festgestellt mittels einer Agar-Verdünnungs-methode, ergibt sich aus der nachstehenden Tabelle:

# 0 003 616

Antimikrobielles Spektrum von Trestatin A, Trestatin B und Trestatin C

| Testorganismus | Trestatin A | Trestatin B | Trestatin C |
|---|---|---|---|
| E. coli NIHJ | >100 $\mu$g/ml | >100 $\mu$g/ml | >100 $\mu$g/ml |
| E. coli NIHJ SMf | >100 | >100 | >100 |
| E. coli NIHJ STf | >100 | >100 | >100 |
| E. coli K–12 MLl630 | >100 | >100 | >100 |
| E. coli CF17 | >100 | >100 | >100 |
| E. coli CF41 | >100 | >100 | >100 |
| Salmonella typhimurium IFO12529 | >100 | >100 | >100 |
| Salmonella paratyphi B | >100 | >100 | >100 |
| Citrobacter | >100 | >100 | >100 |
| Shigella flexneri | >100 | >100 | >100 |
| Klebsiella pneumoniae PCl602 | >100 | >100 | >100 |
| Pseudomonas aeruginosa IFO12689 | >100 | >100 | >100 |
| Pseudomonas aeruginosa A3 | >100 | >100 | >100 |
| Pseudomonas aeruginosa P2 | >100 | >100 | >100 |
| Proteus vulgaris OX19 ATCC6898 | >100 | >100 | >100 |
| Bordetella bronchiseptica Aichi 202 | >100 | >100 | >100 |
| Proteus rettgeri | >100 | >100 | >100 |
| Serratia marcescens IFO12648 | >100 | >100 | >100 |
| Staphylococcus aureus FDA209P | >100 | >100 | >100 |
| Staphylococcus aureus MS3937 | >100 | >100 | >100 |
| Staphylococcus aureus MS9261 | >100 | >100 | >100 |
| Staphylococcus aureus Smith | >100 | >100 | >100 |
| Bacillus subtilis PCI 219 | >100 | >100 | >100 |
| Sarcina lutea ATCC9341 | >100 | >100 | >100 |
| Candida albicans yu1200 | >100 | >100 | >100 |
| Mycobacterium smegmatis ATCC607 | >100 | >100 | >100 |

3) Amylase-Hemmwirkung:

Die Hemmwirkung auf Schweinepankreas $\alpha$-Amylase von Trestatin A, B und C deren Salzen und Gemischen davon ist nachstehend beschrieben. Die Aktivitäten wurden nach der oben erwähnten Methode bestimmt.

| Amylase-Hemmer | Hemmaktivitäten (IU/g) |
|---|---|
| Trestatin Komplex* | $3{,}6 \times 10^7$ |
| Trestatin A | $7{,}1 \times 10^7$ |
| Trestatin B | $1{,}5 \times 10^6$ |
| Trestatin C | $4{,}9 \times 10^7$ |

13

**0 003 616**

| | |
|---|---|
| Trestatin A.HCl | $6,3 \times 10^7$ |
| Trestatin B.HCl | $1,3 \times 10^6$ |
| Trestatin C.HCl | $4,5 \times 10^7$ |

|  | Hemmung der durch gekochte Reisstärke induzierten Hyperglycämie | |
|---|---|---|
|  | Ratten $ED_{20**}$ (mg/kg) | Mäuse p.o. |
| Trestatin A | 1,0 | 1,1 |
| Trestatin B | 7,7 | 3,2 |
| Trestatin C | 3,0 | 0,7 |

\* Trestatin Komplex bezieht sich auf das Präparat das gemäss Beispiel 1, —II Isolierung 5) erhalten wurde, welches Trestatin A, Trestatin B und Trestatin C enthält.

\*\* $ED_{20}$ ist diejenige Dosis die nach 20 Minuten eine 20%ige Abnahme der Hyperglycämie verursacht die durch orale Verabreichung von 2 g/kg Körpergewicht gekochter Reisstärke erzeugt wurde.

Die Trestatine können auch $\alpha$-Amylase aus Bacillus subtilis und Aspergillus oryzae hemmen und Amylo-$\alpha$-1,4-$\alpha$-1,6-glucosidase aus Aspergillus niger. Sie zeigen jedoch keine Hemmwirkung auf $\beta$-Amylase aus Süsskartoffeln.

Die Verdauung der Stärke, des Hauptkohlenhydrats in menschlichen Nahrungsmitteln, beginnt mit der Wirkung der $\alpha$-Amylase. Dieses Enzym findet sich in der Saliva und im Pankreassekret. Die Pankreasamylase ist hauptsächlich verantwortlich für die vollständige Verdauung der Stärke im Gastrointestinal-Trakt. Sie katalysiert das Aufbrechen des Kohlenhydratpolymers, das letztlich in Maltose resultiert, die wiederum durch intestinale Disaccharidasen zu Glucose hydrolysiert wird. Infolgedessen wird kurz nach einer Mahlzeit von stärkehaltigen Lebensmitteln eine ausgeprägte Hyperglycämie die zu einer Hyperinsulinämie führt beobachtet.

Bekanntlich ist bei Diabetikern dieser Hyperglycämie sehr ausgeprägt und höchst unerwünscht. Bei übergewichtigen Menschen ist diese Hyperglycämie oft von einer vermehrten Insulinsekretion begleitet, die zu einer Erschöpfung der $\beta$-Zellen des Pankreas führen kann. Ausserdem begünstigt eine Hyperinsulinämie die Lipogenese, die zur Hyperlipidämie beiträgt.

Trestatin A, B und C Präparate, die eines oder mehrere dieser Trestatine enthalten, können postprandiale Hyperglycämie und Hyperinsulinämie verringern, wie sich bei Experimenten bei Ratten und Mäusen nach Stärkebelastung (Seite 24 und Tab. 1) oder nach Verabreichung von Futter (Tab. 2) zeigt.

Die durch orale Sucrosezufuhr induzierte Reduktion der Hyperglycämie (Tab. 1) zweigt neben der Hemmung der $\alpha$-Amylase eine gewisse Sucrase-Hemmung an. Auf die Glucose-induzierte Hyperglycämie wird kein Effekt ausgeübt.

Die erfindungsgemässen Trestatine sind infolgedessen bei der Behandlung von Diabetes, Fettleibigkeit, Hyperlipidämie und Atheroschlerose von Wert.

Da $\alpha$-Amylase im Speichel durch Verdauung von Stärke im Mund zu Karies beitragen kann, können Trestatine auch zur Verhütung oder Verminderung der Ausbildung von Karies angewandt werden.

Trestatine können weiterhin als biochemische Reagentien verwendet werden.

14

TAB. 1 : Wirkung von Trestatin auf die einer oralen Belastung mit nativer Stärke oder Sucrose folgende Hyperglycämie (Hyperinsulinämie)
(Plasmaglucose und Insulin 20 Minuten nach Kohlenhydraten + Trestatin (1,6 g Weizenstärke/kg oder 3,2 g Sucrose/kg). Die Resultate sind angegeben als Prozentsätze von Kohlenhydrat-belasteten Kontrollen (* (p 0,05), ** (p 0,01), *** (p 0,001) gemäss dem Kolmogorov-Smirnov-Test)

| | Dosis/kg | Stärke-Belastung | | Sucrose-Belastung |
| | | Glucose | Insulin | Glucose |
|---|---|---|---|---|
| Trestatin Gemisch *) | 6 600 IU | 75 *** | 61 * | — |
| | 22 000 IU | 60 *** | 59 * | — |
| | 66 000 IU | 56 *** | 41 ** | — |
| | 220 000 IU | — | — | 90 * |
| | 660 000 IU | — | — | 73 *** |
| | 2 200 000 IU | — | — | 64 *** |
| Trestatin A | 0,3 mg | 68 *** | 66 * | — |
| | 1,0 mg | 56 *** | 42 *** | — |
| | 3,0 mg | 55 *** | 42 *** | — |
| | 30,0 mg | — | — | 84 * |
| Trestatin C | 0,3 mg | 71 ***. | 51 ** | — |
| | 1,0 mg | 60 *** | 51 ** | — |
| | 3,0 mg | 58 *** | 63 ** | — |
| | 30,0 mg | — | — | 85 * |

*) entspricht dem Stufe IV-Pulver das gemass Beispiel 1, −II Isolierung 4) erhalten wurde und das Trestatin A, Trestatin B und Trestatin C enthalt.

TAB.2 : Effekt von Trestatin auf Hyperglycämie und Hyperinsulinämie auf orale Verabreichung von Futter an Ratten.

| Dosis | Plasma-Glucose in mg/100 ml (Mittel ± S.E.) | | | Plasma-Insulin in uU/ml (Mittel ± S.E.) | | |
| | 20 Min. | 60 Min. | 120 Min. | 20 Min. | 60 Min. | 120 Min. |
|---|---|---|---|---|---|---|
| Futter (Kontrolle) | 275 ± 9 | 228 ± 11 | 191 ± 6 | 59 ± 10 | 44 ± 10 | 30 ± 5 |
| Futter + 22 440 IU | 205 ± 9 ** | 190 ± 14 * | 184 ± 4 | 15 ± 2 ** | 18 ± 3 | 17 ± 2 |
| Futter + 67 320 IU | 197 ± 2 ** | 173 ± 5 ** | 175 ± 3 * | 22 ± 8 * | 14 ± 1 ** | 13 ± 1 * |
| Futter + 224 400 IU | 189 ± 5 ** | 164 ± 2 ** | 169 ± 2 * | 18 ± 3 * | 8 ± 1 ** | 9 ± 1 ** |

Gruppe von 6 weiblichen Ratten (85–95 g) fasteten 24 Stunden und erhielten Futter *) durch Magensonde (2,2 g /6 ml/Ratte) ± Trestatin Gemisch **)

*) normales Laborfutter suspendiert in 0,07% Carageenan
**) entsprechend dem Stufe IV-Pluver das gemäss Beispiel 1, II Isolierung 4) erhalten wurde und das Trestatin A, B und C enthält

Statistik gemäss Kolmogorov-Smirnov * (p 0,05), ** (p 0.01), ·** (p 0,001)

Die erfindungsgemässen Trestatine und ihre Salze können als Medikamente z.B. in Form pharmazeutischer Präparate verwendet werden, die sie im Gemisch mit einem zur enteralen Anwendung geeigneten organischen oder anorganischen inerten Trägermaterial enthalten, wie beispielsweise Wasser, Gelatine, Gummi arabicum, Lactose, Stärke, Magnesiumstearat, Talk, vegetative Oele und Polyalkylenglykole, Die pharmazeutischen Präparate können in fester Form z.B. als Tabletten, Dragées oder Kapseln oder in flüssiger Form z.B. als Lösungen oder Suspensionen vorliegen.
Eine Dosiseinheit kann 20—50 mg Wirkstoff enthalten. Die tägliche Dosis an Erwachsenen kann sich zwischen 10 und 200 mg bewegen und ist entsprechend individuellen Erfordernissen zu variieren.

Die Trestatine und ihre Salze können auch als Zusätze zu Nahrungsmitteln und Diätpräparaten wie Zucker, Fruchtsaft, Schokolade, Marmelade, Kartoffelprodukten und Brot verwendet werden. Zweckmässig enthalten solche Nahrungsmittel oder Präparate 0,1 bis 1 Gew.% der erfindungsgemässen Trestatine.

Die folgenden Beispiele erläutern die Erfindung weiter.

## Beispiel 1

I *Fermentation*

Ein Nährmedium wurde aus den folgenden Bestandteilen hergestellt:

| | | |
|---|---|---|
| Kartoffelstärke | 20 | g |
| Glucose | 20 | g |
| Hefeextrakt | 5 | g |
| Natriumchlorid | 2,5 | g |
| Mineralsalzlösung *1 | 1 | ml |
| entionisiertes Wasser | 1 | Liter |

*1 Die Lösung enthält 50 g $ZnSO_4 . 7H_2O$ und je 5 g $CuSO_4 . 5H_2O$ und $MnCl_2 . 4H_2O$ pro Liter entionisiertes Wasser

Das pH der obigen Lösung wurde durch Zusatz von 6N Natriumhydroxyd auf 6 eingestellt, danach wurden 3,2 g Calciumcarbonat und 20 g Sojabohnenmehl zugesetzt und die Nährlösung wurde 20 Minuten bei 120°C dampfsterilisiert.

Sporen von einer Schrägagarkultur von Streptomyces dimorphogenes sp. NR—320—OM7HB (FERM—P No. 3664) wurden auf 10 500 ml Erlenmeyer-Flaschen, die jeweils 110 ml sterilisiertes Medium enthielten, übergeimpft. Die Flaschen wurden auf eine Rotationsschüttelmaschine mit einer Umdrehungszahl von 185/Min. gesetzt und 72 Stunden bei 27°C geschüttelt. Danach wurden die Kulturen zur Beimpfung eines 50 l Fermentationsgefässes verwendet, das 25 l des gleichen Mediums enthielt. Der Fermentationszyklus betrug etwa 43 Stunden, währenddessen die Temperatur auf 27°C gehalten wurde und filtrierte Luft mit einer Geschwindigkeit von 25 l/Min. zugeführt wurde. Die Schüttelgeschwindigkeit betrug 300 Bewegungen/Min. Ein typisches Trestatin-Fermentationsbier (pH 6,9) wies eine Aktivität von $3,1 \times 10^4$ IU/ml auf.

II *Isolierung*

Die in der Fermentationsbrühe enthaltenen Trestatine können durch Adsorption an Aktivkohle und Austauscherharze isoliert werden. Bei dem hierbei erwähnten Austauschermaterial Dowex 50 und Dowex 1 handelt es sich um Handelsprodukte der Firma Dow Chemical, bei Amberlit CG 50 um ein Handelsprodukt der Firma Rohm & Haas Company.

1) *Kohleadsorption:*

Das aus der oben beschriebenen Trestatin-Fermentation erhaltene Vollbier wurde mit 5N Natriumhydroxyd auf pH 7,0 gestellt und dann filtriert. Das Filtrat (19 l, $3,1 \times 10^4$ IU/ml, $5,9 \times 10^8$ IU/total) wurden mit 380 g Aktivkohle versetzt und das Gemisch wurde 20 Minuten bei Raumtemperatur gerührt und dann filtriert. Der Kohlekuchen wurde mit 4 l Leitungswasser gewaschen und in 10 l heissem 50%igem wässrigem Aceton suspendiert. Die Suspension wurde durch Zusatz von 6N HCl auf pH 2 gestellt, 20 Minuten unter Rühren bei 60°C gehalten, wobei das Gemisch durch gelegentlichen Zusatz von 6N HCl auf pH 2 gehalten wurde. Das Gemisch wurde dann filtriert und der Kohlekuchen nochmals mit weiteren 10 l 50%igem wässrigem Aceton in gleicher Weise behandelt. Die vereinigten Filtrate wurden durch Zusatz von 6N NaOH auf pH 7 gestellt und geringe Mengen Niederschlag wurden abfiltriert. Das Filtrat ($5,6 \times 10^8$ IU/total) wurde unter vermindertem Druck auf etwa 3 l konzentriert. Ein Teil des Konzentrats wurde lyophilisiert und lieferte einen rohen Trestatin-Komplex als dunkelbraunes Pulver ($3,2 \times 10^6$ IU/g), der hier als Stufe I-Pulver bezeichnet wird.

2) *Dowex 50 Adsorption:*

Das Konzentrat (etwa 3 l, $5,6 \times 10^8$ IU/total) wurde über eine Säule (80 × 7,5 cm) von Dowex 50 (H-Form, 3,5 l 20—50 mesh) mit einer Geschwindigkeit von etwa 10 ml/Min. gegeben. Die Säule wurde mit 10,5 l entionisiertem Wasser gewaschen, mit 1N Ammoniak eluiert und fraktioniert. Die Fraktionen, die mehr als $3 \times 10^3$ IU/ml Trestatin enthielten, wurden vereinigt, unter vermindertem Druck konzentriert und lyophilisiert und lieferten 75,4 g braunes Pulver ($7,0 \times 10^6$ IU/g, $5,3 \times 10^8$ IU/total), das im folgenden als Stufe II-Pulver bezeichnet wird.

**0 003 616**

3) *Methanol-Behandlung:*

75,4 g Stufe II-Pulver wurde in 3,770 ml Methanol suspendiert und 2 Stunden bei Raumtemperatur gerührt. Der unlösliche Teil wurde filtriert, mit wenig Methanol gewaschen, getrocknet und lieferte 41,5 g braunes Pulver ($1,1 \times 10^7$ IU/g, $4,6 \times 10^8$ IU/total), im folgenden als Stufe III-Pulver bezeichnet.

4) *Dowex 1-Chromatographie:*

41,5 g Stufe III-Pulver wurden in 100 ml entionisiertem Wasser gelöst. Die Lösung wurde auf eine Säule (87 × 5,5 cm) von Dowex 1 (Acetat-Form, 2,050 ml, 200—400 mesh) gegeben. Die Säule wurde mit entionisiertem Wasser mit einer Geschwindigkeit von 2,5 ml/Min. entwickelt und das Eluat in 17 ml Fraktionen fraktioniert. Die aktiven Fraktionen 35—70 wurden vereinigt, konzentriert, unter vermindertem Druck eingedampft und lyophilisiert und lieferten 22,6 g eines gelben Pulvers ($1,8 \times 10^7$ IU/g, $4,1 \times 10^8$ IU/total), im folgenden als Stufe IV-Pulver bezeichnet.

5) *Dowex 50-Chromatographie:*

22,6 g Stufe IV-Pulver wurden in 50 ml entionisiertem Wasser gelöst und die Lösung auf eine Säule (85 × 6,6 cm) von Dowex 50 (Ammonium-Form, 2,900 ml, 200—400 mesh) gegeben. Die Säule wurde mit entionisiertem Wasser eluiert (2,5 ml/Min.) und das Eluat in 17 ml Fraktionen gesammelt. Die aktiven Fraktionen 65—89 wurden vereinigt, konzentriert und lyophilisiert und lieferten 10,6 g Trestatin-Komplex als hellgelbes Pulver ($3,6 \times 10^7$ IU/g, $3,8 \times 10^8$ IU/total), im folgenden als Stufe V-Pulver bezeichnet.

III *Trennung des Trestatin-Komplexes in Trestatin A, B und C*

10,6 g Stufe V-Pulver wurden in 20 ml destilliertem Wasser gelöst und die Lösung wurde auf eine Säule (78 × 4,8 cm) von Amberlit CG 50 (Mischbett bestehend aus 3,5 Teilen Ammonium-Form und 6,5 Teilen H-Form, 1,420 ml, Typ I) gegeben. Die Säule wurde mit destilliertem Wasser mit 2,0 ml/Min. eluiert und das Eluat in 14 ml Fraktionen gesammelt. Die aktiven Fraktionen wurden der Hochdruckflüssigchromatographie unter den nachstehenden Bedingungen unterworfen:

Säule: $\mu$ Bondapak/Carbohydrate (1,4″ × 1′, Waters Associate)

Träger: $CH_3CN{:}H_2O$ (63:37)

Durchflussge-schwindigkeit: 4,0 ml/Min.

Injektionsvolumen: 2—10 $\mu$l

Nachweis: Ultraviolett Absorption bei 210 nm

Die entsprechenden Fraktionen wurden vereinigt, konzentriert und lyophilisiert. Die Resultate sind nachstehend angegeben:

| Fraktion No. | Gewicht des Rückstandes (g) | Aktivität (IU/g) | Hauptkomponente (Retentionszeit) |
|---|---|---|---|
| 71—90 | 1,40 | $9,4 \times 10^6$ | Trestatin B (3,4 Min.) |
| 131—165 | 1,88 | $5,9 \times 10^7$ | Trestatin A (5,5 Min.) |
| 311—380 | 0,79 | $4,4 \times 10^7$ | Trestatin C (8,8 Min.) |

IV *Herstellung von Trestatin A*

Ein reines Trestatin A-Präparat wurde durch Rechromatographie auf Amberlit CG 50 hergestellt. 1,17 g des gemäss Abschnitt III erhaltenen Trestatin A wurden in 4 ml destilliertem Wasser gelöst und auf eine Säule (133 × 1,7 cm) von Amberlit CG 50 (Mischbett bestehend aus 8 Teilen H-Form und 2 Teilen Ammonium-Form (300 ml, Typ II) gegeben. Die Säule wurde mit destilliertem Wasser eluiert und das Eluat in 7 ml Fraktionen gesammelt, wobei die einzelnen Fraktionen durch Hochdruckflüssigchromatographie, wie oben beschrieben, überwacht wurden. Die Fraktionen 245—465 enthielten Trestatin A und wurden vereinigt, konzentriert und lyophilisiert und lieferten 649 mg Trestatin A

17

als weisses Pulver mit den oben angegebenen Eigenschaften.

V *Herstellung von Trestatin B*

Ein reines Trestatin B-Präparat wurde durch Rechromatographie auf Amberlit CG 50 erhalten. 450 mg von gemäss Abschnitt III erhaltenen Trestatin B wurden in 2 ml destilliertem Wasser gelöst und auf eine Säule (122 × 1,7 cm) von Amberlit CG 50 (Mischbett, bestehend aus 8 Teilen H-Form und 2 Teilen Ammonium-Form, 280 ml, Typ II) gegeben. Die Säule wurde mit destilliertem Wasser eluiert und das Eluat in 3 ml-Fraktionen gesammelt und durch Hochdruckflüssigchromatographie, wie oben beschrieben, überwacht. Die Fraktionen 121—129 enthielten Trestatin B und wurden vereinigt, konzentriert und lyophilisiert. Man erhielt 170 mg Trestatin B als weisses Pulver von den oben angegebenen Eigenschaften.

VI *Herstellung von Trestatin C*

Ein reines Trestatin C-Präparat wurde durch Rechromatographie auf Amberlit CG 50 erhalten. 720 mg von gemäss Abschnitt III erhaltenen Trestatin C wurden in 3 ml destilliertem Wasser gelöst und auf eine Säule (133 × 1,7 cm) von Amberlit CG 50 (Mischbett, bestehend aus 6,2 Teilen H-Form und 3,8 Teilen Ammonium-Form, 300 ml, Typ II) gegeben. Die Säule wurde mit destilliertem Wasser eluiert und das Eluat in 6 ml-Fraktionen gesammelt und durch Hochdruckflüssigchromatographie, wie oben beschrieben, überwacht. Die Fraktionen 42—59 enthielten Trestatin C und wurden vereinigt, konzentriert und lyophilisiert. Man erhielt 515 mg von Trestatin C als weisses Pulver mit den oben beschriebenen Eigenschaften.

Beispiel 2
Herstellung von Trestatin A-hydrochlorid

95 mg Trestatin A wurden in 1 ml destilliertem Wasser gelöst und das pH durch Zusatz von 0,1N Salzsäure auf pH 2,0 gestellt. Die Lösung wurde unter vermindertem Druck auf ein Volumen von etwa 0,5 ml konzentriert. Danach wurden 6 ml Aethanol zugegeben und der gebildete weisse Niederschlag wurde durch Zentrifugieren gesammelt, mit 2 ml Aethanol gewaschen und unter vermindertem Druck getrocknet. Man erhielt 97 mg Trestatin A-dihydrochlorid als weisses Pulver.

Beispiel 3
Herstellung von Trestatin B-hydrochlorid

92 mg des gemäss Beispiel 1 erhaltenen Trestatin B wurden in 1 ml destilliertem Wasser gelöst und die Lösung mit 0,1N Salzsäure auf pH 2,0 eingestellt. Die Lösung wurde unter vermindertem Druck auf etwa 0,5 ml eingeengt und mit 7 ml Aethanol versetzt. Der Niederschlag wurde abzentrifugiert, mit 2 ml Aethanol gewaschen und getrocknet und lieferte 91 mg Trestatin B-monohydrochlorid als weisses Pulver.

Beispiel 4
Herstellung von Trestatin C-hydrochlorid

90 mg des gemäss Beispiel 1 hergestellten Trestatin C wurden in 1 ml destilliertem Wasser gelöst und die Lösung mit 0,1N Salzsäure auf pH 2,0 eingestellt. Die Lösung wurde auf 0,5 ml eingeengt, mit 6 ml Aethanol versetzt, abzentrifugiert, der Niederschlag mit 2 ml Aethanol gewaschen und getrocknet. Man erhielt 91 mg Trestatin C-trihydrochlorid als weisses Pulver.

Beispiel 5

In Analogie zu den in Beispiel 1 beschriebenen Verfahren wurde unter Verwendung von Streptomyces dimorphogenes sp. NR—320—OM7HBS (FERM—P No. 3665) Trestatin A, Trestatin B und Trestatin C erhalten.

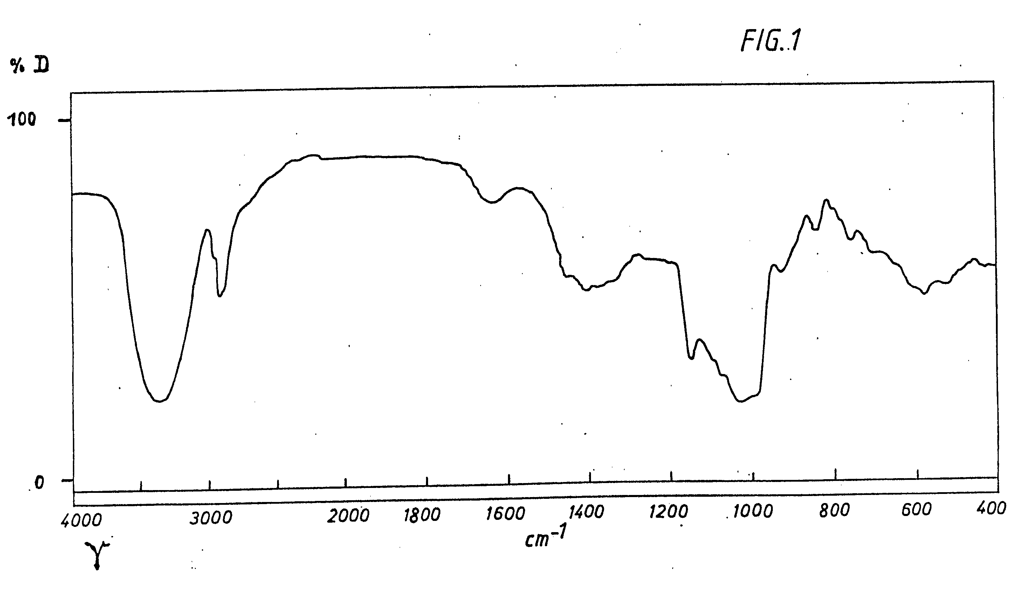

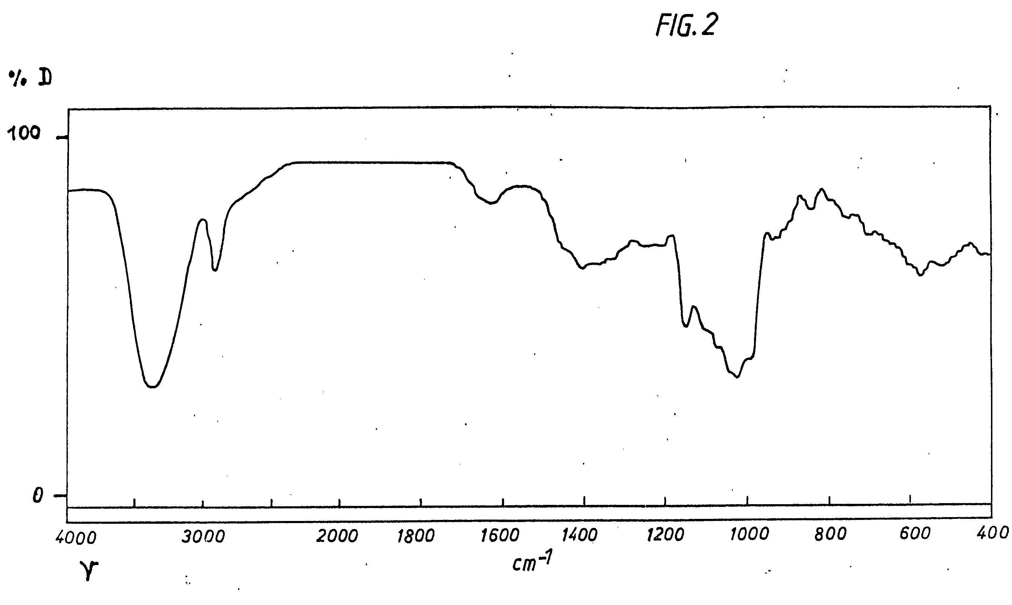

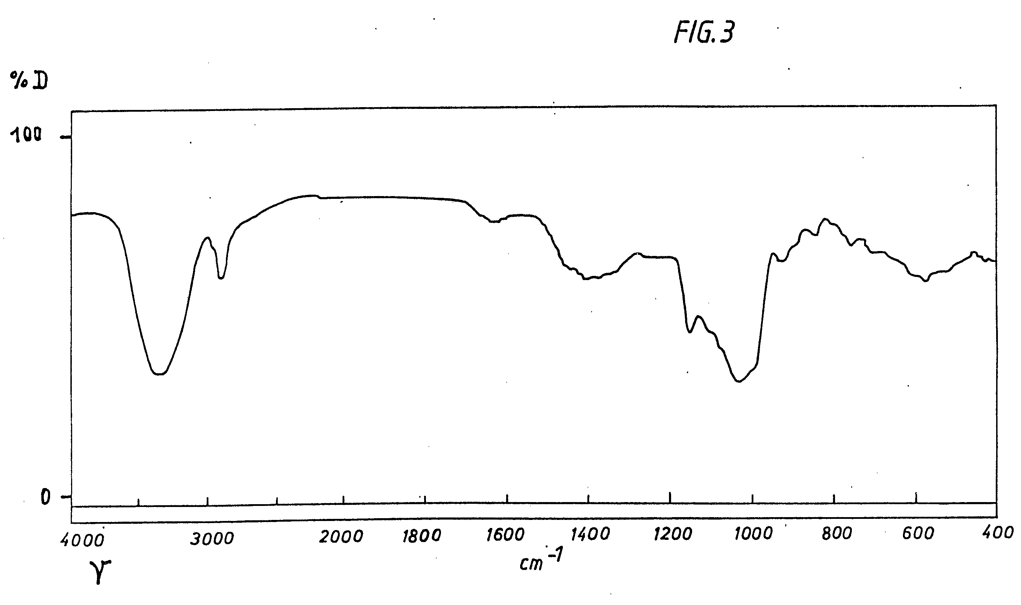

Die Figuren 1, 2 und 3 stellen Infrat-Absorptionsspektrum von Trestatin A, Trestatin B und Trestatin C dar.

Die Figuren 4, 5 und 6 zeigen das $^1$H NMR-Spektrum in $D_2O$ bei 100 MHz von Trestatin A, Trestatin B und Trestatin C.

Die Figur 7 zeigt das Hochdruckflüssigchromatogramm der Trestatin A, B und C.

**Patentansprüche**

1. Die Aminozuckerderivate Trestatin A, Trestatin B und Trestatin C, deren Salze und Gemische davon, gekennzeichnet durch Trehalose als gemeinsames Strukturmerkmal und durch die folgenden Eigenschaften:

a) Elementar-Analyse:

|  | C % | H % | N % | O % |
|---|---|---|---|---|
| Trestatin A: | 46,72 | 7,13 | 2,08 | 43,28 |
| Trestatin B: | 46,27 | 7,04 | 1,65 | 44,69 |
| Trestatin C: | 47,55 | 7,15 | 2,29 | 42,66 |

b) Molekulargewicht (Osmometrie):

| Trestatin A: | 1470 |
|---|---|
| Trestatin B: | 975 |
| Trestatin C: | 1890 |

c) Schmelzpunkt:

| Trestatin A: | 221—232°C (Zers.) |
|---|---|
| Trestatin B: | 209—219°C (Zers.) |
| Trestatin C: | 230—237°C (Zers.) |

d) Spezifische Drehung:

| Trestatin A: | $[\alpha]_D^{24} = +177°$ (c = 1,0, $H_2O$) |
|---|---|
| Trestatin B: | $[\alpha]_D^{26} = +187°$ (c = 1,0, $H_2O$) |
| Trestatin C: | $[\alpha]_D^{23} = +169,5°$ (c = 1,0, $H_2O$) |

e) Ultraviolett-Absorptionsspektrum (in Wasser):

Trestatin A, Trestatin B und Trestatin C zeigen Endabsorption.

f) Infrarot-Absorptionsspektrum (in KBr):

| Trestatin A: | siehe Figur 1 |
|---|---|
| Trestatin B: | siehe Figur 2 |
| Trestatin C: | siehe Figur 3 |

g) $^1$H NMR-Spektrum (in $D_2O$ bei 100 MHz):

| Trestatin A: | siehe Figur 4 |
|---|---|
| Trestatin B: | siehe Figur 5 |
| Trestatin C: | siehe Figur 6 |

h) Löslichkeit in Lösungsmitteln:

Trestatin A, Trestatin B und Trestatin C und deren Hydrochloride sind leicht löslich in Wasser; löslich in Dimethylsulfoxyd; schwach löslich in Aethanol und Aceton; und unlöslich in Aethylacetat und Chloroform;

i) Farbreaktion:

Trestatin A, Trestatin B und Trestatin C geben eine positive Permanganat und Anthron-Reaktion und eine negative Sakaguchi-Reaktion.

2. Amylase-hemmende Mittel, gekennzeichnet durch einen Gehalt an Trestatin A, Trestatin B oder Trestatin C, deren Salzen oder Gemischen davon.

3. Verfahren zur Herstellung von Trestatin A, Trestatin B und Trestatin C, deren Salzen oder Gemischen davon, dadurch gekennzeichnet, dass man Streptomyces dimorphogenes NR—320—OM7HB (=ATCC 31484) oder NR—320—OM7HBS (=ATCC 31485) unter aeroben Bedingungen in wässrigem Medium kultiviert, die Trestatine aus der Fermentationsbrühe abtrennt und gewünschtenfalls Trestatin A, Trestatin B und Trestatin C voneinander trennt.

**Revendications**

1. Les dérivés aminés de sucre Trestatine A, Trestatine B et Trestatine C, leurs sels et leurs mélanges, caractérisés par du tréhalose comme particularité structurale commune et par les propriété suivantes:

a) Analyse élémentaire:

|              | C %   | H %  | N %  | O %   |
|--------------|-------|------|------|-------|
| Trestatine A:| 46,72 | 7,13 | 2,08 | 43,28 |
| Trestatine B:| 46,27 | 7,04 | 1,65 | 44,69 |
| Trestatine C:| 47,55 | 7,15 | 2,29 | 42,66 |

b) Masse moléculaire (osmométrie):

| Trestatine A: | 1470 |
|---------------|------|
| Trestatine B: | 975  |
| Trestatine C: | 1890 |

c) Point de fusion:

| Trestatine A: | 221—232°C (déc.) |
|---------------|-------------------|
| Trestatine B: | 209—219°C (déc.) |
| Trestatine C: | 230—237°C (déc.) |

d) Rotation spécifique:

| Trestatine A: | $[\alpha]_D^{24} = +177°$ (c = 1,0, $H_2O$) |
|---------------|---------------------------------------------|
| Trestatine B: | $[\alpha]_D^{26} = +187°$ (c = 1,0, $H_2O$) |
| Trestatine C: | $[\alpha]_D^{23} = +169,5°$ (c = 1,0, $H_2O$) |

e) Spectre d'absorption dans l'ultraviolet (dans l'eau):
La trestatine A, la Trestatine B et la Trestatine C présentent une absorption terminale.

f) Spectre d'absorption dans l'infrarouge (dans KBr):

| Trestatine A: | voir figure 1 |
|---------------|---------------|
| Trestatine B: | voir figure 2 |
| Trestatine C: | voir figure 3 |

**0 003 616**

g) Spectre de RMN ¹H (dans $D_2O$ à 100 MHz):

    Trestatine A:           voir figure 4

    Trestatine B:           voir figure 5

    Trestatine C:           voir figure 6

h) Solubilité dans les solvants:

    La Trestatine A, la Trestatine B et la Trestatine C ainsi que leurs chlorhydrates sont facilement solubles dans l'eau; solubles dans le diméthylsulfoxyde; faiblement solubles dans l'éthanol et l'acétone; et insolubles dans l'acétate d'éthyle et le chloroforme.

i) Réactions de coloration:

    La Trestatine A, la Trestatine B et la Trestatine C sont positives aux réactions du permanganate et de l'anthrone et négatives à la réaction de Sakaguchi

    2. Agent inhibiteur d'amylase, caractérisé par une teneur en Trestatine A, Trestatine B ou Trestatine C, leurs sels ou leurs mélanges.

    3. Procédé pour la production de Trestatine A, de Trestatine B et de Trestatine C, de leurs sels ou de leurs mélanges, caractérisé en ce qu'on cultive Streptomyces dimorphogenes NR—320—OM7HB (=ATCC 31484) ou NR—320—OM7HBS (=ATCC 31485) dans des conditions aérobies dans un milieu aqueux, on recueille les Trestatines à partir du bouillon de fermentation et, si nécessaire, on sépare la Trestatine A, la Trestatine B et la Trestatine C les unes des autres.

**Claims**

1. The amino sugar derivatives Trestatin A, Trestatin B and Trestatin C, their salts and mixtures thereof, characterized by having trehalose as a common structural feature and by having the following properties:

a) Elementary analysis:

| | C % | H % | N % | O % |
|---|---|---|---|---|
| Trestatin A: | 46.72 | 7.13 | 2.08 | 43.28 |
| Trestatin B: | 46.27 | 7.04 | 1.65 | 44.69 |
| Trestatin C: | 47.55 | 7.15 | 2.29 | 42.66 |

b) Molecular weight (osmometry):

| | |
|---|---|
| Trestatin A: | 1470 |
| Trestatin B: | 925 |
| Trestatin C: | 1890 |

c) Melting point:

| | |
|---|---|
| Trestatin A: | 221—232°C (dec.) |
| Trestatin B: | 209—219°C (dec.) |
| Trestatin C: | 230—237°C (dec.) |

d) Specific rotation:

Trestatin A:    $[\alpha]_D^{24} = +177°$ (c = 1.0, $H_2O$)

Trestatin B:    $[\alpha]_D^{26} = +187°$ (c = 1.0, $H_2O$)

Trestatin C:    $[\alpha]_D^{23} = +169.5°$ (c = 1.0, $H_2O$)

**0 003 616**

e) Ultraviolet absorption spectrum (in water):

Trestatin A, Trestatin B and Trestatin C show end absorption.

f) Infrared absorption spectrum (in KBr):

| Trestatin A: | see Figure 1 |
| Trestatin B: | see Figure 2 |
| Trestatin C: | see Figure 3 |

g) $^1$H NMR spectrum (in $D_2O$ at 100 MHz)

| Trestatin A: | see Figure 4 |
| Trestatin B: | see Figure 5 |
| Trestatin C: | see Figure 6 |

h) Solubility in solvents:

Trestatin A, Trestatin B and Trestatin C and their hydrochlorides are readily soluble in water; soluble in dimethyl sulphoxide; slightly soluble in ethanol and acetone; and insoluble in ethyl acetate and chloroform.

i) Colour reaction:

Trestatin A, Trestatin B and Trestatin C give a positive permanganate and anthrone reaction and a negative Sakaguchi reaction.

2. An amylase-inhibiting composition, characterized by a content of Trestatin A, Trestatin B or Trestatin C, their salts or mixtures thereof.

3. A process for the manufacture of Trestatin A, Trestatin B and Trestatin C, their salts or mixtures thereof, characterized by cultivating Streptomyces dimorphogenes NR—320—OM7HB (= ATCC 31484) or NR—320—OM7HBS (= ATCC 31485) under aerobic conditions in an aqueous medium, separating the Trestatins from the fermentation broth and, if desired, separating, Trestatin A, Trestatin B and Trestatin C from one another.

22

FIG.1

0003616

FIG. 2

0003 616

FIG.3

%D

100

0

cm$^{-1}$

4000  3000  2000  1800  1600  1400  1200  1000  800  600  400

3

FIG.4

0003 616

DSS

HDO

PPM

$\delta_H$

FIG.5

0 003 616

FIG. 6

HDO

SB.

DSS

10 9 8 7 6 5 4 3 2 1 0

PPM

δ_H

0003 616

FIG.7

TRESTATIN B

TRESTATIN A

TRESTATIN C

RELATIVE ABSORPTION

0.05

0.025

0

0          5          10

RETENTIONS ZEIT (MIN.)

0          5

RETENTIONS ZEIT (MIN.)

0          5

RETENTIONS ZEIT (MIN.)

0003616

FIG.8 ANGENOMMENE STRUKTUR FÜR TRESTATIN *A* UND *B*

TRESTATIN *A*

TRESTATIN *B*